# EUROPÄISCHE PATENTSCHRIFT

(11) **EP 2 158 935 B1**
(45) Veröffentlichungstag und Bekanntmachung des Hinweises auf die Patenterteilung: **16.07.2014**
(21) Anmeldenummer: 08163270.5
(22) Anmeldetag: 29.08.2008
(51) Int. Cl.: A61M 39/10, F16L 37/092, A61M 39/12

(54) **Steckverbinder für medizinische Schläuche**
Connector for medicinal tubes
Connecteur à fiche pour tuyaux médicaux

(43) Veröffentlichungstag der Anmeldung: 03.03.2010
(73) Patentinhaber: Dräger Medical GmbH, 23558 Lübeck (DE)
(72) Erfinder: Dr. Grassl, Thomas, 23560, Lübeck (DE); Dr. Koulechov, Kirill, 23669, Timmendorfer-Strand (DE)
(74) Vertreter: UEXKÜLL & STOLBERG

(56) Entgegenhaltungen:
- EP-A- 1 143 185
- EP-A- 1 636 521
- WO-A1-2005/037362
- US-A- 5 549 865
- US-B1- 6 334 634

## Beschreibung

Die vorliegende Erfindung betrifft einen Steckverbinder für medizinische Schläuche gemäß dem Anspruch 1.

Medizinische Steckverbinder werden beispielsweise zur Verbindung einer Blutdruckmanschette mit einer Blutdruckauswerteeinheit in der Anwendung einer nicht-invasiven Blutdruckmessung verwendet.

Aus der US-Patentschrift 3,640,552 ist ein Steckverbinder für Druck- oder Vakuumschläuche bekannt. Der bekannte Verbinder weist ein inneres Steckerteil und ein äußeres Steckerteil auf, das in zusammengesetztem Zustand einen vorderen Endbereich des inneren Steckerteils umgreift und mit vorstehenden elastischen Einrasthaken mit radial nach innen vorragenden Vorsprüngen versehen ist, die beim Aufschieben auf das innere Steckerteil zunächst radial nach außen gebogen werden und dann durch Einrasten in entsprechende Vertiefungen in der Außenfläche des inneren Steckerteils eine Verbindung der beiden Steckerteile miteinander bewirken. Das elastische Einrasten der Einrasthaken mit ihren Vorsprüngen in den Vertiefungen bewirkt eine axiale Fixierung der beiden Steckerteile zueinander. Ein umlaufender Dichtungsvorsprung ist in dem Inneren des Durchgangs als frei vorstehender Teil des inneren Steckerteils so vorgesehen, dass er beim Einrasten der Einrasthaken in Anlage an einen Dichtungsvorsprung im inneren Durchgang des äußeren Steckerteils kommt und durch Andruck eine Dichtung zwischen den Steckerteilen herstellt. Die durch den Steckverbinder zu verbindenden Schläuche sind jeweils in das von dem Verbindungsbereich abgewandten Ende der Steckerteile eingeführt und in deren Inneren fixiert, wobei die Schläuche nicht bis in den Verbindungsbereich der beiden Steckerteile hineinreichen. Es sind jeweils vom Aufbau her komplizierte Abdichtungen von jedem Schlauch zum jeweiligen Steckerteil und der beiden Steckerteile untereinander vorhanden.

Ein Steckverbinder für medizinische Schläuche ist aus EP 1 584 348 B1 bekannt, der ebenfalls ein inneres und ein äußeres Steckerteil aufweist. Das äußere Steckerteil hat an seinem den Verbindungsbereich zugewandten Ende einen als Hohlzylinder ausgebildeten Eintrittsabschnitt und axial daran anschließend eine radial nach außen erweiterten abgerundeten Halteabschnitt, der zum formschlüssigen Eingriff mit einem nach außen vorstehenden Halteabschnitt an dem inneren Steckerteil ausgebildet ist. Der Halteabschnitt an dem inneren Steckerteil ist so ausgestaltet, dass er beim Zusammenstecken und Lösen der Steckerteile unter elastischer Verformung eines oder beider Steckerteile durch den Eintrittsabschnitt in dem äußeren Steckerteil hindurchgleiten kann, wonach er nach Erreichen des radial erweiterten Halteabschnitts an der Innenfläche des äußeren Steckerteils einen Eingriff mit elastischem Formschluss erzeugt, wodurch gleichzeitig eine Halte- und eine Dichtfunktion durch die ineinandergreifenden Halteabschnitte bewirkt werden. Die durch den Steckverbinder zu verbindenden Schläuche sind jeweils auf an den von dem Verbindungsbereich abgewandten Enden vorgesehenen Anschlussstutzen von außen aufgeschoben. Dadurch muss im Bereich dieser Anschlussstutzen jeweils eine weitere abgedichtete Verbindung zwischen dem jeweiligen Schlauch und dem äußeren Ende des Steckerteils vorgesehen sein. Dies macht den Aufbau des Steckverbinders insgesamt kompliziert, da sowohl zwischen den Schläuchen und dem jeweiligen Steckerteil als auch zwischen den jeweiligen Steckerteilen selbst dichte Verbindungen hergestellt werden müssen. Die zentrale Durchgangsöffnung der jeweiligen Steckverbinder weist einen unterschiedlichen Durchmesser auf, wobei insbesondere im Kupplungsbereich der Innendurchmesser größer ausgebildet ist im Vergleich zu dem kupplungsfernen Bereich der Steckverbinder. Verschiedene Durchmesser der Durchgangsöffnung der Steckverbinder können bei der Übertragung des Messsignals insbesondere bei einer nicht-invasiven Blutdruckmessung zu einer Messsignaldämpfung und damit zu einem ungenauen Messergebnis führen.

Aus EP 1636 521 B1 eine Verbindungseinrichtung für eine. Steckverbindung eines Rohres mit einem Kupplungskörper bekannt. Die Verbindungseinrichtung weist einen Kupplungskörper und eine Klemmhülse auf. Im zusammengesteckten Zustand umgreift der vordere Endbereich des Kupplungskörpers die Klemmhülse. Dabei ist am vorderen Ende des Kupplungskörpers ein nach innen ragender Vorsprung vorhanden, der zum Eingriff mit einer Ausnehmung in der Außenfläche des ersten Steckerteils vorgesehen ist, wobei zwischen dem Kupplungakörper und dem Klemmkörper in axialer Richtung Spiel vorhanden ist.

Aus WO 2005/037362 A1 ist ein Konnektor für medizinische Flüssigkeiten enthaltende Verpackungen mit zwei miteinander verrasteten Steckerteilen bekannt; der Konnektor weist die Merkmale des Oberbegriffs von Anspruch 1 auf. Im Inneren liegt zwischen zwei Schultern der Steckerteile eine selbstabdichtende Membran. Nach Abbrechen eines Teils des äu-ßeren Steckerteils liegt die Membran frei und kann durch den Kegelschaft einer eingeführten Spritze durchdrungen werden, wobei die selbstabdichtende Membran den Kegelschaft dann gegenüber dem Außenraum, in dem der Rest der Spritze liegt abdichtet.

Aufgabe der vorliegenden Erfindung ist es, die im Stand der Technik aufgezeigten Nachteile zu überwinden und einen Steckverbinder zu schaffen, der sowohl in der Herstellung als auch in der Handhabung einfach ist und eine sichere Verbindung der Steckerteile gewährleistet.

Zur Lösung dieser Aufgabe dienen die kennzeichnenden Merkmale des Patentanspruchs 1. Vorteilhafte Ausführungsformen der Erfindung sind in den Unteransprüchen angegeben.

Erfindungsgemäß ist ein Steckverbinder für medizinische Schläuche vorgesehen, der ein erstes und ein zweites Steckerteil umfasst, wobei im zusammengesteckten Zustand das zweite Steckerteil im vorderen Endbereich das erste Steckerteil umgreift, wobei das zweite Steckerteil wenigstens ein nach vorn vorstehendes Einrastelement aufweist, welches an seinem vorderen Ende wenigstens einen radial nach innen ragenden Vorsprung aufweist, der zum Eingriff in eine komplementäre Ausnehmung in der Außenfläche des ersten Steckerteils vorgesehen ist, um so durch Eingriff des Vorsprungs in die Ausnehmung das zweite Steckerteil in axialer Richtung fixiert auf dem ersten Steckerteil zu halten. Ferner weisen beide Steckerteile jeweils einen zentralen Durchgang auf. Ein Steckerteil hat in einem Verbindungsbereich einen sich zur Ausgangsöffnung hin aufweitenden Endbereich und das andere Steckerteil ist mit einem koaxial in seinem Inneren angeordneten elastischen Teilstückfest verbunden, das in den Verbindungsbereich vorragt, so dass das vordere Ende des elastischen Teilstücks in Anlage an den aufgeweiteten Endbereich des gegenüberliegenden Steckerteils gedrückt ist, wenn der Vorsprung des wenigstens einen Einrastelements das zweite Steckerteil in Eingriff mit dem ersten Steckerteil hält, so dass das elastische Teilstück durch den Andruck an den sich aufweitenden Endbereich eine Dichtungsfunktion ausübt. Durch diese Anpressung des vorderen Endbereichs des elastischen Teilstücks an die Innenfläche des gegenüberliegenden, sich aufweitenden Endbereiches wird durch elastische Anpressung und Verformung des Endes des elastischen Teilstücks eine Dichtung geschaffen. Das elastische Teilstück kann als ein elastisches Schlauchstück ausgebildet sein. Das elastische Schlauchstück kann ein separates Schlauchteil sein. In einer bevorzugten Ausführungsform ist das elastische Schlauchstück jedoch das vordere Ende des von hinten in das zugehörige Steckerteil eingeführten Schlauchs, der in fester, ganzflächiger Verbindung mit der Innenfläche des Steckerteils ist. In dieser Ausgestaltung der erfindungsgemäßen Steckverbindung ist es möglich, dass der Schlauch mit einem der Steckerteile integriert ist, so dass hier keine weitere Abdichtung an diesem Steckerteil notwendig ist, und gleichzeitig eine Dichtungsfunktion der Steckerteile untereinander bewirkt.

Auf diese Weise wird eine einfach lösbare und sicherere Steckverbindung geschaffen.

Das elastische Teilstück, vorzugsweise das elastische Schlauchstück oder der Schlauch, kann zum Beispiel in den inneren Durchgang des Steckerteils eingeklebt sein. Alternativ kann das elastische Teilstück in einem Stück mit dem Steckerteil hergestellt sein, beispielsweise durch eine Zwei-Komponenten-Spritzgussverfahrensweise, wobei dabei die Härte (Shore-Härte) des elastischen Teilstücks geringer ausgeführt ist als die sich anschließende Steckerkomponente. Bevorzugt weist das elastische Teilstück eine Shore-Härte von 60 bis 80 ShA auf.

Zur Erzielung einer guten Dichtung ist der aufgeweitete Endbereich des Steckerteils konusförmig ausgebildet. Alternativ kann der aufgeweitete Endbereich des Steckerteils auch kugelförmig ausgebildet sein.

In vorteilhafter Weise ist der Innendurchmesser des elastischen Teilstücks des zweiten Steckerteils gleich dem Innendurchmesser des inneren Durchgangs des ersten Steckerteils und dem Innendurchmesser eines Anschlussschlauches. Das daraus resultierende gleichbleibende Lumen gewährleistet eine verlustarme Schallwellendurchleitung in der Anwendung des Steckverbinders bei einem nicht-invasiven Blutdruckmesssystem. Es kann hierbei eine bessere Signalqualität erzielt werden und somit ein Blutdruck eines Patienten exakter detektiert werden. Eine Anwendung des erfindungsgemäßen Steckverbinders für ein nicht-invasives Blutdruckmesssystem ermöglicht somit eine Erhöhung der Patientensicherheit.

Die Erfindung wird im Folgenden anhand eines Ausführungsbeispiels in den Zeichnungen beschrieben, in denen:
- Figur 1: eine Draufsicht auf eine erste Ausführungsform der Steckverbindung zeigt;
- Figur 2: einen Schnitt durch den Steckverbinder entlang der Ebene A-A aus Figur 1 zeigt;
- Figur 3: einen vergrößerten Ausschnitt aus Figur 2 zeigt, der in Figur 2 mit einem Kreis umrandet ist;
- Figur 4: eine perspektivische Ansicht des Steckverbinders der ersten Ausführungsform ist;
- Figur 5: eine Detailansicht des in Figur 4 mit einem Kreis umrandeten Ausschnitts zeigt;
- Figur 6: einen Schnitt entsprechend Figur 2 eines Steckverbinders gemäß einer zweiten Ausführungsform zeigt; und
- Figur 7: eine vergrößerte Detailansicht des in Figur 6 mit einem Kreis umrandeten Ausschnitts zeigt.

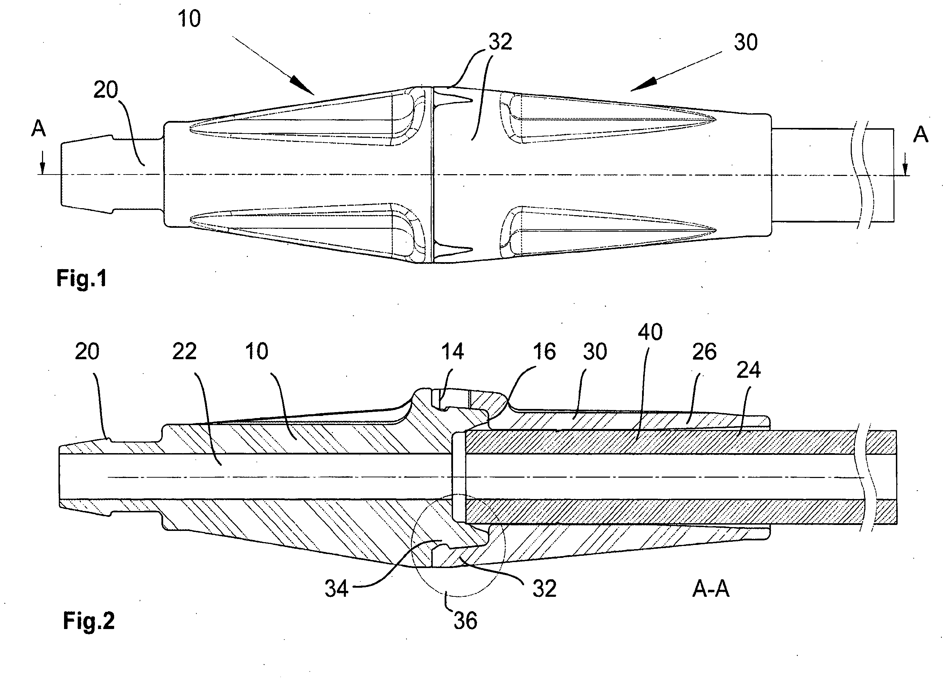

Der in den Figuren 1 und 2 gezeigte Steckverbinder hat ein erstes Steckerteil 10 und ein zweites Steckerteil 30, das mit seinem vorderen Ende die Außenfläche des ersten Steckerteils 10 umgreift. Das einem Verbindungsbereich 36 zugewandte Ende des zweiten Steckerteils 30 hat als Einrasthaken ausgebildete Einrastelemente 32, die in axialer Richtung nach vorn in dem Verbindungsbereich 36 vorstehen und an ihrem vorstehenden Ende radial nach innen weisende Vorsprünge 34 haben. Die Vorsprünge 34 sind zum Eingriff in eine komplementäre Ausnehmung 14 an der Außenfläche des ersten Steckerteils 10 vorgesehen. Die Vorsprünge 34 gleiten beim Ineinanderschieben der Steckerteile 10 und 30 auf der vorderen Außenfläche des vorderen Endes des ersten Steckerteils 10, die sich in diesem Bereich leicht konisch aufweitet, wodurch die Einrastelemente 32 elastisch nach außen gebogen werden. Wenn die Vorsprünge 34 der Einrastelemente 32 die Ausnehmung 14 in der Außenfläche des ersten Steckerteils 10 erreichen, werden sie elastisch dort hinein gedrückt, um so das zweite Steckerteil 30 an dem ersten Steckerteil 10 zu fixieren.

In dem inneren Durchgang 26 des zweiten Steckerteils 30 ist ein elastisches Teilstück 40 integriert. Das elastische Teilstück 40 ist als elastischen Schlauchstück ausgebildet, wobei das elastische Teilstück 40 in diesem Ausführungsbeispiel in die innere Durchgangsöffnung 26 des zweiten Steckerteils 30 eingeklebt ist. Alternativ kann das elastische Teilstück 40 in einem Stück mit dem zweiten Steckerteil 30 hergestellt sein, wobei das Material des elastischen Teilstücks 40 vorzugsweise mit einer geringeren Shore-Härte als das zweite Steckerteil 30 hergestellt ist. Das elastische Teilstück 40 weist in vorteilhafter Weise eine Shore-Härte von 60 bis 80 ShA auf. Das Material des elastischen Teilstücks 40 kann in dieser Ausführung aus thermoplastischem Elastomer oder thermoplastischem Polyurethan bestehen. In einer weiteren Ausführungsvariante kann einer der beiden Steckerteile 10 oder 30 aus einem elastischen Material mit einer geringeren Shore-Härte als das andere Steckerteil 10 oder 30 hergestellt sein.

Das dem Verbindungsbereich 36 des ersten Steckerteils 10 entgegen gesetzte Ende weist einen Befestigungsstutzen 20 zum Aufschieben eines Schlauches auf. In vorteilhafter Weise ist der Innendurchmesser eines inneren Durchgangs 24 des in dem zweiten Steckerteil 30 angeordneten elastischen Teilstücks 40 gleich dem Innendurchmesser eines inneren Durchgangs 22 des ersten Steckerteils 10. Der Innendurchmesser der inneren Durchgänge 22 und 24 der beiden Steckerteile 10 und 30 entsprechen dem Innendurchmesser des an dem Befestigungsstutzen 20 anzuschließenden Schlauches. Das daraus resultierende gleichbleibende Lumen gewährleistet eine verlustarme Schallwellendurchleitung in der Anwendung des Steckverbinders bei einem nicht-invasiven Blutdruckmesssystem.

Das vordere Ende des in dem zweiten Steckerteil 30 befestigten elastischen Teilstücks 40 ragt über das vordere Ende des zweiten Steckerteils 30 hinaus. Der dem Verbindungsbereich 36 zugewandte Endbereich des ersten Steckerteils 10 weist einen sich axial auf den Verbindungsbereich 36 zu konisch aufweitenden Endbereich 16 auf, wobei der Innendurchmesser des aufgeweiteten Endbereichs 16 am äußersten Ende größer als des Außendurchmesser des vorstehenden elastischen Teilstücks 40 ist und der Innendurchmesser der konisch aufgeweiteten Endbereichs 16 weiter im Inneren des inneren Durchgangs 22 des ersten Steckerteils 10 kleiner als der Außendurchmesser des elastischen Teilstücks 40 ist. Dadurch wird das vordere Ende des elastischen Teilstücks 40 in der in den Figuren 1 bis 3 dargestellten eingerasteten Stellung der Einrastelemente 32 gegen die Innenfläche des konisch aufgeweiteten Endbereichs 16 gedrückt, wodurch eine ringförmig umlaufende Andruckfläche gebildet wird, die eine Dichtungsfunktion ausübt, um so den inneren Durchgang 22 des ersten Steckerteils 10 mit dem inneren Durchgang 24 des elastischen Teilstücks 40 abgedichtet zu verbinden. Alternativ kann der aufgeweitete Endbereich 16 des ersten Steckerteils 10 auch kugelförmig ausgebildet sein.

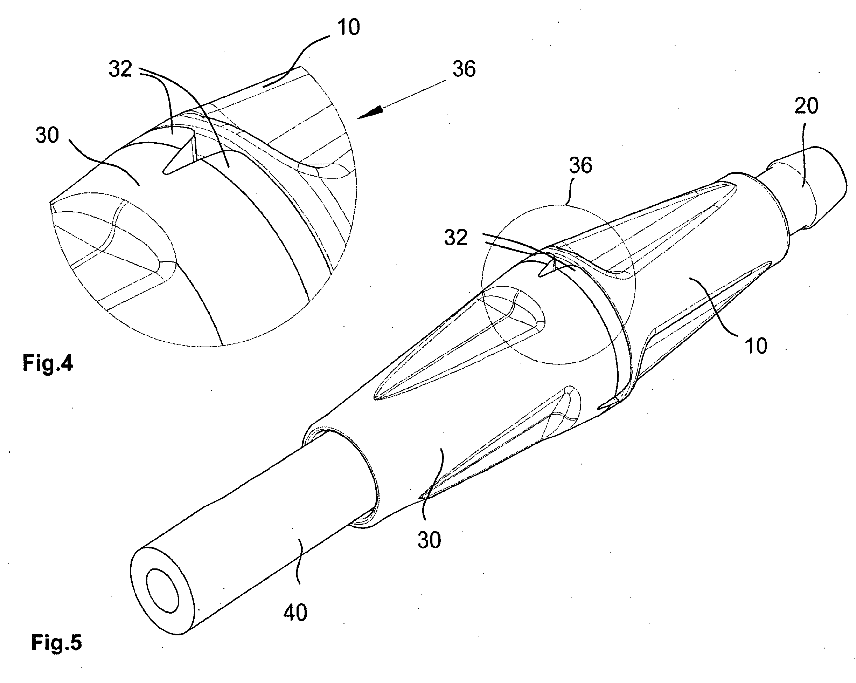

Figuren 4 und 5 zeigen die Steckverbindung in zusammengestecktem Zustand in perspektivischer Ansicht. Das zweite Steckerteil 30 ist mit seinem integrierten elastischen Teilstück 40 versehen, welches wieder als elastisches Schlauchstück ausgebildet ist. Das dem Verbindungsbereich 36 des ersten Steckerteils 10 entgegen gesetzte Ende weist einen Befestigungsstutzen 20 zum Aufschieben eines Schlauches auf. In der perspektivischen Darstellung wird deutlich, dass die Einrastelemente 32 zylindersegmentförmige Bereiche des vorderen Endes des zweiten Steckteils 30 sind, in diesem Fall drei Zylindersegmente, die einen Umfangswinkel von beinahe 120° einnehmen und jeweils nur durch kleine Einschnitte in dem vorderen Endbereich des zweiten Steckteils 30 voneinander getrennt sind.

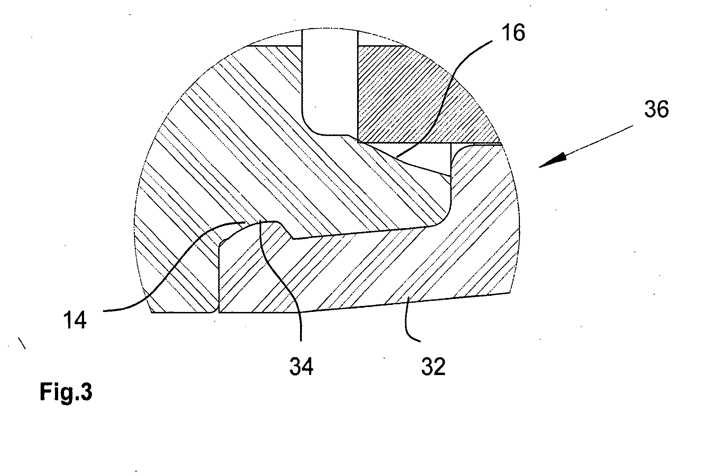

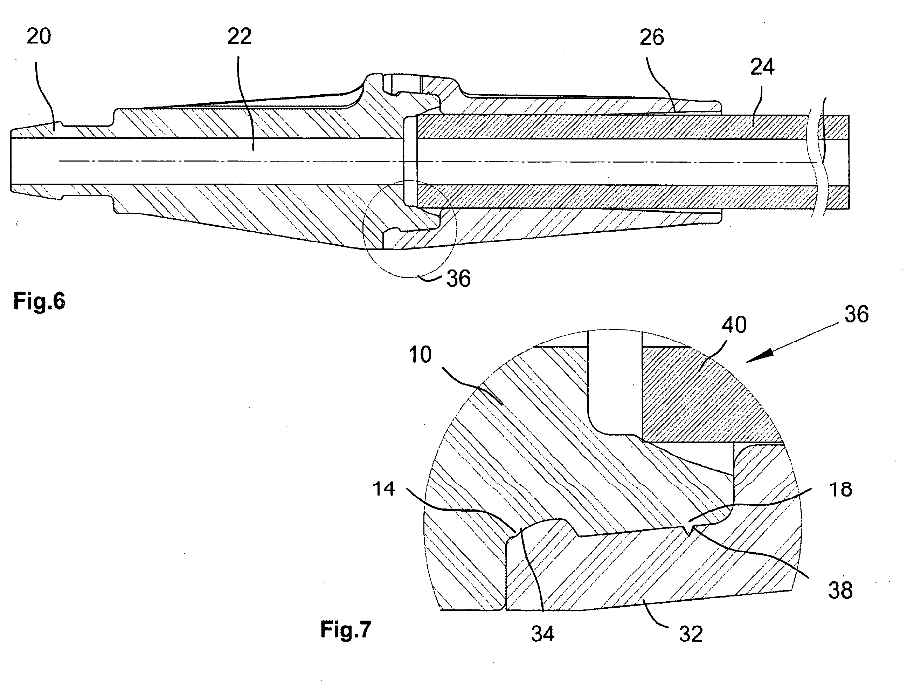

Figuren 6 und 7 zeigen Figuren 2 und 3 entsprechende Ansichten in einer zweiten Ausführungsform, die in allen Merkmalen dem ersten Ausführungsbeispiel entspricht, jedoch zusätzlich eine umlaufende Nut 38 (siehe Figur 7) an der Innenfläche der Einrastelement 32 aufweist. An der Außenfläche des vorderen Endbereichs des ersten Steckteils 10 ist ein zu der umlaufenden Nut 38 komplementärer Grat 18 gebildet. Der formschlüssige Eingriff des Grats 18 mit der Nut 38 sorgt für eine weitere Einrastung und Erhöhung der Haltekraft der Steckverbindung. Zum Lösen der Verbindung muss eine Zugkraft auf die Steckerteile 10, 30 axial weg voneinander ausgeübt werden, wobei bei Überschreiten einer Schwellenkraft eine hinreichende elastische Verformung der Einrastelement 32 und des vorderen Endbereichs des ersten Steckerteils 10 auftritt, die zum Lösen der Einrastverbindung führt.

In den dargestellten Ausführungsformen ist das elastische Teilstück 40 in das zweite Steckerteil 30 integriert. Umgekehrt ist es aber auch möglich, das elastische Teilstück 40 in dem ersten Steckerteil 10 vorzusehen und die gegenüberliegende konische Aufweitung zum Anpressen des Endes des elastischen Teilstücks 40 in dem zweiten Steckerteil 30 anzuordnen.

### BEZUGSZEICHENLISTE

- 10: erstes Steckerteil
- 14: Ausnehmung
- 16: Endbereich
- 18: Grat
- 20: Befestigungsstutzen
- 22: innerer Durchgang des ersten Steckerteils 10
- 24: innerer Durchgang des elastischen Teilstücks 40
- 26: innere Durchgangsöffnung des zweiten Steckerteils 30
- 30: zweite Steckerteile
- 32: Einrastelement
- 34: Vorsprung
- 36: Verbindungsbereich
- 38: Nut
- 40: elastisches Teilstück

## Patentansprüche

1. Steckverbinder für medizinische Schläuche
mit einem ersten Steckerteil (10) und einem zweiten Steckerteil (30), wobei im zusammengesteckten Zustand das zweite Steckerteil (30) im vorderen Endbereich das erste Steckerteil (10) umgreift,
wobei das zweite Steckerteil (30) wenigstens ein nach vorn vorstehendes Einrastelement (32) aufweist, welches an seinem vorderen Ende wenigstens einen nach innen ragenden Vorsprung (34) aufweist, der zum Eingriff in eine komplementäre Ausnehmung (14) in der Außenfläche des ersten Steckerteils (10) vorgesehen ist, um so durch Eingriff des Vorsprungs (34) in die Ausnehmung (14) das zweite Steckerteil (30) in axialer Richtung fixiert auf dem ersten Steckerteil (10) zu halten,
wobei beide Steckerteile (10, 30) jeweils einen zentralen Durchgang (22, 24) aufweisen,
wobei ein Steckerteil (10, 30) in seinem Verbindungsbereich (36) einen sich zur Ausgangsöffnung hin aufweitenden Endbereich (16) hat,
**dadurch gekennzeichnet, dass**
das andere Steckerteil (10, 30) mit einem in seinem Inneren angeordneten elastischen Teilstück (40) mittels Kleben oder durch einstückige Herstellung fest verbunden ist, das in den Verbindungsbereich (36) vorragt, so dass das vordere. Ende des elastischen Teilstücks (40) in Anlage an den aufgeweiteten Endbereich (16) des gegenüberliegenden Steckerteils (10, 30) gedrückt ist, wenn der Vorsprung (34) des wenigstens einen Einrastelements (32) das zweite Steckerteil (30) in Eingriff mit dem ersten Steckerteil (10) hält, so dass das elastische Teilstück (40) durch den Andruck an den sich aufweitenden Endbereich (16) eine Dichtungsfunktion ausübt, durch die die zentralen Durchgänge (22, 24) abgedichtet verbunden werden.

2. Steckverbinder nach Anspruch 1, **dadurch gekennzeichnet, dass** das elastische Teilstück (40) in das erste oder zweite Steckerteil (10, 30) eingeklebt ist.

3. Steckverbinder nach einem der Ansprüche 1 und 2, **dadurch gekennzeichnet, dass** das elastische Teilstück (40) als ein elastisches Schlauchstück ausgebildet ist.

4. Steckverbinder nach Anspruch 3, **dadurch gekennzeichnet, dass** das elastische Schlauchstück das vordere Ende des mit dem ersten oder zweiten Steckerteils (10, 30) verbundenen Schlauches darstellt.

5. Steckverbinder nach Anspruch 1, **dadurch gekennzeichnet, dass** das elastische Teilstück (40) in einem Stück mit einem der beiden Steckerteile (10, 30),hergestellt ist, wobei das Material des elastischen Teilstücks (40) vorzugsweise mit einer geringeren Shore-Härte als das Steckerteil (10, 30) hergestellt ist.

6. Steckverbinder nach Anspruch 5, **dadurch gekennzeichnet, dass** das elastische Teilstück (40) aus einem thermoplastischen Elastomer oder einem thermoplastischen Polyurethan besteht.

7. Steckverbinder nach einem der vorhergehenden Ansprüche, **dadurch gekennzeichnet, dass** eines der beiden Steckerteile (10, 30) aus einem elastischen Material mit einer geringeren Shore-Härte als das andere Steckerteil (10, 30) hergestellt ist.

8. Steckverbinder nach einem der vorhergehenden Ansprüche, **dadurch gekennzeichnet, dass** der aufgeweitete Endbereich (16) konisch oder kugelförmig ausgebildet ist.

9. Steckverbinder nach einem der vorhergehenden Ansprüche, **dadurch gekennzeichnet, dass** zwei oder mehr Einrastelemente (32) vorgesehen sind, die als zylindersegmentförmige Fortsätze des zweiten Steckerteils (30) ausgebildet sind, die durch Einschnitte in die Außenwand des vorderen Endes des zweiten Steckerteils (30) voneinander getrennt sind.

10. Steckverbinder nach einem der vorhergehenden Ansprüche, **dadurch gekennzeichnet, dass** der Innendurchmesser des elastischen Teilstücks (40) gleich dem Innendurchmesser des anschließenden Durchgangs der beiden Steckerteile (10, 30) ist.

11. Steckverbinder nach einem der vorhergehenden Ansprüche, **dadurch gekennzeichnet, dass** eine umlaufende Nut (38) an der Innenfläche der Einrastelemente (32) und an der Außenfläche des vorderen Endbereichs des ersten Steckerteils (10) und ein zu der umlaufenden Nut (38) komplementärer Grat (18) ausgebildet ist, wobei die Vorsprünge (34) der Einrastelemente (32) beim Einrasten in die Ausnehmungen (14) ebenfalls in formschlüssigem Eingriff einrasten.

12. Steckverbinder nach einem der vorhergehenden Ansprüche in Kombination mit einem System zur nicht-invasiven Blutdruckmessung eines Probanden umfassend wenigstens:
eine Blutdruckmanschette zur Erfassung des Blutdrucks,
und einen Monitor zur Auswertung und Anzeige des Blutdrucks des Probanden.

13. Verwendung eines Steckverbinders gemäß den Ansprüchen 1 bis 11 in einem nicht-invasiven Blutdruckmesssystem.

## Claims

1. A plug-in connector for medical tubes,
comprising a first plug-in part (10) and a second plug-in part (30) wherein the second plug-in part (30) in a plugged-together state embraces in a front end portion the first plug-in part (10),
wherein the second plug-in part (30) has at least one forwardly protruding snap-in element (32) which has at a front end at least one inwardly protruding projection (34) which is provided for engaging a complementary recess (14) in the outer surface of the first plug-in part (10) in order to hold the second plug-in part (30) fixed in an axial direction on the first plug-in part (10) due to the projection (34) meshing with the recess (14),
wherein each of both plug-in parts (10, 30) has a central passage (22, 24),
wherein one of the plug-in parts (10, 30) has in its connection area (36) an end area (16) expanding towards the outlet opening,
**characterized in that**
the other of the plug-in parts (10, 30) is firmly connected by gluing or integral forming to an elastic section (40) being disposed in its interior, which elastic section protrudes into the connection area (36), such that a front end of the elastic section (40) is pressed into contact with the expanded end area (16) of the opposite one of the plug-in parts (10, 30) when the projection (34) of the at least one snap-in element (32) maintains the second plug-in part (30) meshed with the first plug-in part (10), so that the elastic section (40) by pressing on the expanding end area (16) exerts a sealing function by the which the central passages (22, 24) are connected in a sealed manner.

2. The plug-in connector according to claim 1, **characterized in that** the elastic section (40) is bonded into the first or second plug-in part (10, 30).

3. The plug-in connector according to claims 1 and 2, **characterized in that** the elastic section (40) is formed by an elastic tube section.

4. The plug-in connector according to claim 3, **characterized in that** the elastic tube section is formed by a front end of the tube connected to the first or second plug-in part (10, 30).

5. The plug-in connector according to claim 1, **characterized in that** the elastic section (40) is made in one piece with one of the plug-in parts (10, 30), wherein the material of the elastic section (40) is preferably produced with a lower Shore hardness than the plug-in part (10, 30).

6. The plug-in connector according to claim 5, **characterized in that** the elastic section (40) consists of a thermoplastic elastomer or a thermoplastic polyurethane.

7. The plug-in connector according to any of the preceding claims, **characterized in that** one of the two plug-in parts (10, 30) is manufactured from an elastic material with a lower Shore hardness than the other plug-in part (10, 30).

8. The plug-in connector according to any of the preceding claims, **characterized in that** the expanding end area (16) is designed conical or spherical.

9. The plug-in connector according to any of the preceding claims, **characterized in that** two or more snap-in elements (32) are provided, which are formed as extensions of the second plug-in part (30) and have the shape of cylinder segments which are separated from each other by incisions in the outer wall of the front end of the second plug-in part (30).

10. The plug-in connector according to any of the preceding claims, **characterized in that** the inner diameter of the elastic section (40) is equal to the inner diameter of the adjoining passage of both plug-in parts (10, 30).

11. The plug-in connector according to any of the preceding claims, **characterized in that** a circumferential groove (38) is formed on the inner surface of the snap-in elements (32), and that a burr (18) complementary to the circumferential groove (38) is formed on the outer surface of the front end area of the first plug-in part (10), wherein the projections (34) of the snap-in elements (32) during snapping-in snap in the recesses (14) in a positive-locking manner.

12. The plug-in connector according to any of the preceding claims in combination with a system for non-invasive blood pressure measurement of a test person, the system at least comprising:
a blood pressure cuff for detecting the blood pressure,
and a monitor for analysis and display of the blood pressure of the test person.

13. Use of a plug-in connector according to claims 1 to 11 in a non-invasive blood pressure measuring system.

## Revendications

1. Connecteur à fiche pour tuyaux médicaux,
comprenant un premier élément de connecteur (10) et un deuxième élément de connecteur (30), sachant qu'à l'état enfiché, le deuxième élément de connecteur (30) entoure le premier élément de connecteur (10) dans la partie d'extrémité avant,
le deuxième élément de connecteur (30) présentant au moins un élément d'encliquetage (32) qui dépasse vers l'avant et comporte à son extrémité avant au moins une saillie (34) qui s'étend vers l'intérieur et est prévue pour entrer en prise dans un évidement (14) complémentaire dans la surface extérieure du premier élément de connecteur (10), afin de maintenir ainsi, par l'entrée en prise de la saillie (34) dans l'évidement (14), le deuxième élément de connecteur (30) sur le premier élément de connecteur (10), en le fixant dans la direction axiale,
les deux éléments de connecteur (10, 30) présentant chacun un passage (22, 24) central,
un élément de connecteur (10, 30) comportant, dans sa zone de connexion (36), une partie d'extrémité (16) qui s'évase en direction de l'ouverture de sortie,
**caractérisé en ce que**
l'autre élément de connecteur (10, 30) est lié solidement, par collage ou fabrication d'une seule pièce, à un tronçon (40) élastique qui est disposé à l'intérieur et avance dans la zone de connexion (36), de sorte que l'extrémité avant du tronçon (40) élastique est appliquée pour être en appui contre la partie d'extrémité (16) évasée de l'élément de connecteur (10, 30) situé en vis-à-vis, lorsque la saillie (34) de l'élément d'encliquetage (32), au nombre d'au moins un, maintient le deuxième élément de connecteur (30) en prise avec le premier élément de connecteur (10), de sorte que le tronçon (40) élastique, du fait de l'application contre la partie d'extrémité (16) évasée, exerce une fonction de joint d'étanchéité, par laquelle les passages (22, 24) centraux sont reliés de façon étanche.

2. Connecteur à fiche selon la revendication 1, **caractérisé en ce que** le tronçon (40) élastique est collé dans le premier ou le deuxième élément de connecteur (10, 30).

3. Connecteur à fiche selon l'une des revendications 1 et 2, **caractérisé en ce que** le tronçon (40) élastique est réalisé sous forme de tronçon de tuyau élastique.

4. Connecteur à fiche selon la revendication 3, **caractérisé en ce que** le tronçon de tuyau élastique constitue l'extrémité avant du tuyau relié au premier ou au deuxième élément de connecteur (10, 30).

5. Connecteur à fiche selon la revendication 1, **caractérisé en ce que** le tronçon (40) élastique est fabriqué d'une seule pièce avec l'un des deux éléments de connecteur (10, 30), le matériau du tronçon (40) élastique étant de préférence fabriqué avec une dureté Shore plus faible que l'élément de connecteur (10, 30).

6. Connecteur à fiche selon la revendication 5; **caractérisé en ce que** le tronçon (40) élastique est constitué d'un élastomère thermoplastique ou d'un polyuréthane thermoplastique.

7. Connecteur à fiche selon l'une des revendications précédentes, **caractérisé en ce que** l'un des deux éléments de connecteur (10, 30) est fabriqué dans un matériau élastique avec une dureté Shore plus faible que l'autre élément de connecteur (10, 30).

8. Connecteur à fiche selon l'une des revendications précédentes, **caractérisé en ce que** la partie d'extrémité (16) évasée est réalisée sous une forme conique ou sphérique.

9. Connecteur à fiche selon l'une des revendications précédentes, **caractérisé en ce que** deux ou plus de deux éléments d'encliquetage (32) sont prévus, qui sont réalisés comme prolongements en forme de segments cylindriques du deuxième élément de connecteur (30), lesquels sont séparés l'un de l'autre par des entailles dans la paroi extérieure de l'extrémité avant du deuxième élément de connecteur (30).

10. Connecteur à fiche selon l'une des revendications précédentes, **caractérisé en ce que** le diamètre intérieur du tronçon (40) élastique est égal au diamètre intérieur du passage des deux éléments de connecteur (10, 30) qui se raccorde.

11. Connecteur à fiche selon l'une des revendications précédentes, **caractérisé en ce qu'**une rainure (38) périphérique est réalisée sur la surface intérieure des éléments d'encliquetage (32) et sur la surface extérieure de la partie d'extrémité avant du premier élément de connecteur (10) et une arête (18), complémentaire de la rainure périphérique, est réalisée, les saillies (34) des éléments d'encliquetage (32) s'enclenchant également pour établir une prise par complémentarité de formes, lors de l'encliquetage dans les évidements (14).

12. Connecteur à fiche selon l'une des revendications précédentes, en combinaison avec un système de mesure non invasive de la tension artérielle d'une personne subissant un examen, comprenant au moins :
un brassard de tension artérielle destiné à détecter la tension artérielle,
et un écran destiné à l'exploitation et à l'affichage de la tension artérielle de la personne subissant l'examen.

13. Utilisation d'un connecteur à fiche selon les revendications 1 à 11 dans un système non invasif de mesure de la tension artérielle.
